# FASCICULE DE BREVET EUROPEEN

(11) **EP 4 637 780 B1**
(45) Date de publication et mention de la délivrance du brevet: **02.12.2026**
(21) Numéro de dépôt: 23841295.1
(22) Date de dépôt: 21.12.2023
(51) Int. Cl.: A61K 31/56, A61K 31/57, A61K 31/575, A61K 31/58, A61P 11/00, A61P 11/06, A61P 11/08, A61K 36/28, A61K 36/88, A61K 36/185, A61K 36/21

(54) **PHYTOECDYSONES POUR LEUR UTILISATION DANS LE TRAITEMENT DE PATHOLOGIES RESPIRATOIRES INFLAMMATOIRES**
PHYTOECDYSONE ZUR VERWENDUNG BEI DER BEHANDLUNG VON ENTZÜNDLICHEN ATEMWEGSERKRANKUNGEN
PHYTOECDYSONES FOR USE IN THE TREATMENT OF INFLAMMATORY RESPIRATORY DISEASES

(30) Priorité: 23.12.2022 FR 2214426
(43) Date de publication de la demande: 29.10.2025
(73) Titulaire: Biophytis, 75001 Paris (FR)
(72) Inventeur: LATIL, Mathilde, 75019 Paris (FR); DILDA, Pierre, 75016 Paris (FR); VEILLET, Stanislas, 91600 Savigny sur Orge (FR)
(74) Mandataire: Santarelli
(86) Numéro de dépôt international: PCT/FR2023/052080
(87) Numéro de publication internationale: WO 2024/134105

(56) Documents cités:
- WO-A1-2020/187679
- WO-A1-2021/198588
- CN-A- 1 279 951
- DINAN LAURENCE ET AL: "20-Hydroxyecdysone, from Plant Extracts to Clinical Use: Therapeutic Potential for the Treatment of Neuromuscular, Cardio-Metabolic and Respiratory Diseases", vol. 9, no. 5, 29 April 2021 (2021-04-29), pages 492, XP093053507, Retrieved from the Internet <URL:https://www.ncbi.nlm.nih.gov/pmc/articles/PMC8146789/pdf/biomedicines-09-00492.pdf> DOI: 10.3390/biomedicines9050492
- SONG G ET AL.: "Protective Effect of 20-Hydroxyecdysterone against Lipopolysaccharides- Induced Acute Lung Injury in Mice", JOURNAL OF PHARMACEUTICS AND DRUG RESEARCH, 7 May 2019 (2019-05-07), pages 109 - 114, XP055651419, Retrieved from the Internet <URL:https://www.scitcentral.com/download.php?journal=14&article=675&file=documents/c70f569e94b539e50e708f26779de9e4.pdf> [retrieved on 20191210]
- DATIR S B ET AL: "EVALUATION OF ANTIALLERGIC ACTIVITY OF THE VARIOUS EXTRACTS OF THE AERIAL PART OF ACHYRANTHES ASPERA VAR. PORPHYRISTACHYA (WALL. EX MOQ.) HOOK.F", PHARMACOLOGYONLINE, 1 January 2009 (2009-01-01), pages 921 - 925, XP093053557, Retrieved from the Internet <URL:https://typeset.io/pdf/evaluation-of-antiallergic-activity-of-the-various-extracts-56sx7a5z2s.pdf> [retrieved on 20230612]

## Description

### Domaine technique de l'invention

L'invention relève des traitements de pathologies respiratoires inflammatoires. Elle s'inscrit dans le domaine des médicaments/compléments alimentaires pour de tels traitements et concerne plus particulièrement les phytoecdysones destinées à la prévention et/ou au traitement de pathologies respiratoires inflammatoires.

### Technique antérieure

Les phytoecdysones représentent une importante famille de phytostérols poly-hydroxylés structuralement apparentés aux hormones de mue des insectes. Ces molécules sont produites par de nombreuses espèces végétales et participent à leur défense contre les insectes ravageurs. La phytoecdysone majoritaire est la 20-hydroxyecdysone (20E). La 20E est pharmacologiquement active chez les mammifères. Elle active le récepteur Mas, un récepteur du bras protecteur du système Rénine Angiotensine (Lafont et al., 2021) qui induit un certain nombre d'effets bénéfiques ayant pu être décrits lors d'études précliniques dans des contextes normaux et pathologiques.

BIO101 est une préparation orale de 20-hydroxyecdysone de pureté supérieure ou égale à 97%. Son procédé de préparation est divulgué dans la demande de brevet internationale WO2018197731 (Lafont et al. 2018). BIO101 est un nouveau candidat médicament développé cliniquement dans le traitement de la sarcopénie, dans la dystrophie musculaire de Duchenne et dans la COVID-19. Ces deux dernières applications thérapeutiques font l'objet de demandes de brevets internationales WO2018197708 (Dilda et al. 2018) et WO2021198588 (Dilda et al. 2021). Des dérivés hémi-synthétiques de 20-hydroxyecdysone ont également été développés, comme divulgué dans la demande de brevet internationale WO2015177469 (Lafont et al. 2015), et sont utilisés pour de telles applications thérapeutiques.

L'asthme est une pathologie inflammatoire chronique des voies respiratoires caractérisé par une inflammation chronique dérégulée, et une réactivité bronchique exacerbée. Il s'agit de la maladie respiratoire la plus fréquente dans le monde, dont la prévalence a considérablement augmenté lors des dernières décennies, atteignant 5 à 10 %, et touchant 339 millions de personnes dans le monde (Bloom et al., 2019 ; Global burden of 369 diseases and injuries in 204 countries and territories, 1990-2019 : a systematic analysis for the Global Burden of Disease Study 2019*).* L'asthme est défini par les symptômes respiratoires variables tels que l'essoufflement, une gêne respiratoire (dyspnée), une respiration sifflante, une oppression thoracique et de la toux, associée à une inflammation bronchique chronique (Redell et al., 2015). La réduction du débit d'air et la présence de mucus entrainent de réelles difficultés à respirer. La fréquence des crises (espacées de quelques heures ou quelques jours, voire de plusieurs mois) et leur sévérité varient d'un patient à l'autre. Les symptômes peuvent s'aggraver lors d'un effort physique ou pendant la nuit.

Certains facteurs de risques sont impliqués dans la survenue d'un asthme qui est l'association d'une prédisposition génétique et de facteurs environnementaux tels que les allergènes présents à l'intérieur des habitations (acariens, moisissures), les allergènes extérieurs (pollens), la fumée de cigarette, ou encore la pollution de l'air (particules fines).

Les caractéristiques physiopathologiques de l'asthme peuvent être divisées en trois composantes interdépendantes : une réactivité bronchique exacerbée des voies respiratoires, le remodelage des voies respiratoires et une inflammation des voies respiratoires.

Les cellules du muscle lisse des voies respiratoires contribuent à la pathogenèse de l'asthme, principalement par leurs propriétés contractiles (Mims, 2015). En effet, la réactivité bronchique exacerbée des voies respiratoires est définie par une réponse exagérée à des stimuli inoffensifs ou nocifs. Cette réponse altérée des bronches à l'environnement dépend de l'activité du muscle lisse bronchique, acteur principal de la bronchoconstriction. Le degré de réactivité accrue des voies respiratoires est en corrélation avec la gravité de l'asthme et la nécessité d'un traitement.

Au-delà des considérations contractiles, l'implication active du muscle lisse des voies respiratoires dans les mécanismes de remodelage bronchique est désormais établie (Boulet, 2018). Le remodelage des voies respiratoires inclut des altérations de l'épithélium avec une hyperplasie des cellules à mucus et une fragilité des cellules épithéliales, une fibrose sous-épithéliale avec modifications de la matrice extracellulaire, une hypertrophie des cellules musculaires lisses bronchiques et une hyperplasie des vaisseaux sanguins, des nerfs et des glandes de la sous-muqueuse bronchique. Les cytokines et les facteurs de croissance, comme le FGF ou le TGFβ, mais aussi la composition de la matrice extracellulaire ont été décrits comme de puissants modificateurs du phénotype des cellules du muscle lisse des voies respiratoires. Ces anomalies sont associées à la chronicité et à la sévérité de l'asthme.

Concernant l'inflammation des voies respiratoires, deux voies physiopathologiques principales sont communément admises : l'asthme éosinophile (ou asthme de type 2) et l'asthme non-éosinophile. Dans l'asthme de type 2, les principales cytokines impliquées sont l'interleukine 4, impliquée dans la polarisation des lymphocytes T auxiliaires 2 et le *switch* vers les immunoglobines E (IgE), l'interleukine 5 associée à la production et au trafic des éosinophiles et enfin, l'interleukine 13 qui joue un rôle central dans le remodelage des voies respiratoires. A l'inverse, l'asthme non-éosinophilique, reste mal compris (Habib et al., 2022 ; Carr et al., 2018).

La majorité des patients asthmatiques sont bien contrôlés par des traitements bêta-agonistes de courte ou longue durée ainsi que par des corticoïdes inhalés, des antileucotriènes, des anticholinergiques, la théophylline ou des biothérapies telles que les anti-lgE (omalizumab), les anti-IL-5 (mepolizumab, reslizumab, et benralizumab) ou encore l'anti-récepteur à l'IL-4 (dupilumab).

Les traitements conventionnels de l'asthme ciblent principalement deux mécanismes physiopathologiques : l'inflammation et la bronchoconstriction. Pour exemples, les bronchodilatateurs inhalés (bêta-2 agonistes et anticholinergiques) ciblent directement les cellules musculaires lisses des voies respiratoires en diminuant leur contractilité afin d'améliorer le débit aérien et de limiter les symptômes chroniques et aigus. Les corticoïdes inhalés quant à eux ciblent principalement l'inflammation mais affectent également la contractilité ainsi que la prolifération des cellules musculaires lisses des voies respiratoires (Goldsmith et al., 2007 ; Goto et al., 2008).

Cependant, l'asthme sévère, le degré le plus grave de la maladie, est non-contrôlé en termes de symptômes chroniques, d'exacerbations, d'obstruction bronchique permanente, qui persistent malgré une prise en charge maximale, qui nécessite un recours continu aux bronchodilatateurs de courte durée d'action malgré des doses maximales de corticoïdes inhalés. Cela conduit les patients à une mauvaise qualité de vie ainsi qu'à des frais de soins directs (visites de soins et traitement), ainsi qu'à des coûts indirects (journée de travail) importants.

Bien que les récentes biothérapies aient considérablement amélioré le cours de la pathologie chez les patients asthmatiques sévères, certains endotypes inflammatoires, notamment l'asthme non-éosinophile, restent orphelins de tout traitement efficace (Ortega et al., 2015 ; Holgate et al., 2004 ; Castro et al., 2018).

La thermoplastie bronchique est une innovation thérapeutique concernant la prise en charge de l'asthme. Il s'agit d'un traitement réalisé par voie endoscopique, efficace de l'asthme modéré à sévère, permettant de diminuer la masse de muscles lisses par l'administration de radiofréquence dans les bronches (Thomson et al., 2012). Bien qu'efficace, cette technique possède des effets secondaires à court à long termes et reste une pratique très invasive.

Il est acquis que l'asthme sévère représente la forme clinique pour laquelle l'inflammation et le remodelage des bronches s'intriquent. Ainsi, le développement de nouveaux traitements agissant sur ces mécanismes représente un défi de santé publique majeur dans ce sens qu'ils pourraient permettre d'améliorer de la gestion thérapeutique des patients atteints et de modifier le pronostic de la maladie (Chanez et al., 2007).

Le muscle lisse des voies respiratoires est réparti au niveau de l'arbre trachéobronchique depuis la trachée jusqu'aux bronchioles terminales. L'innervation cholinergique constitue la commande broncho-constrictrice principale grâce à l'acétylcholine qui permet la contraction du muscle bronchique par son interaction avec des récepteurs muscariniques dont les sous-types M2 et M3 sont présents sur la membrane des cellules musculaires lisses bronchiques humaines (Kolahian and Gosens, 2012).

Néanmoins, la contraction du muscle lisse des voies respiratoires peut être provoquée par un grand nombre de molécules endogènes, telles qu'entre autres l'acétylcholine, l'histamine, les leucotriènes, la bradykinine, l'endothéline-1, l'angiotensine II, ou encore la sérotonine ou 5-Hydroxytryptamine (5-HT) (Bossé, 2012).

La 5-HT est l'un neurotransmetteur du système nerveux central (SNC) les plus étudiés, connu pour avoir des fonctions physiologiques très diverses, en dehors du SNC, telles que la stimulation de la production de cytokines, la vasoconstriction, la prolifération de cellulaire (fibroblastes, cellules musculaires lisses, cellules endothéliales), ou encore la migration des cellules inflammatoires (Ménard et al., 2007 ; Müller et al., 2009 ; Delaney et al., 2011 ; Pakala et al., 1997 ; Boehme et al., 2004 ; Kushnir-Sukhov et al., 2006 ; Filip and Bader, 2009). Dans les poumons, elle semble être impliquée dans les maladies inflammatoires chroniques (Nichols and Nichols, 2008).

La 5-HT exerce ses effets en se liant aux récepteurs cellulaires qui sont classés en sept familles distinctes (5-HT1 à 5-HT7) comprenant 14 sous-types, en fonction de leur diversité structurelle et de leur mode d'action (Kitson, 2007). Les effets de la 5-HT sur les cellules inflammatoires sont principalement assurés par un ou plusieurs des récepteurs suivants : 5-HT1A, 5-HT2A, 5-HT3, 5-HT4 et 5-HT7.

Au-delà de ses fonctions décrites ci-dessus, de nombreuses études ont démontré un rôle de la 5-HT dans la pathogénèse de l'allergie et de l'asthme, en particulier dans la promotion du recrutement des éosinophiles, de l'inflammation des voies respiratoires, d'une réactivité bronchique exacerbée, et du remodelage, induits par les allergènes (Boehme et al., 2004 ; De Bie et al., 1998 ; Lima et al., 2007), caractéristiques de l'asthme allergique.

Il a été démontré que des taux élevés de sérotonine circulante étaient présents chez les patients asthmatiques et également que ces taux de 5-HT étaient corrélés positivement avec l'état clinique des patients et négativement avec la fonction pulmonaire, suggérant que la 5-HT pourrait jouer un rôle dans la physiopathologie de l'asthme aigu (Lechin et al., 1994 ; Lechin et al., 1996).

Au niveau des muscles lisses respiratoires, la 5-HT active à la fois les récepteurs 5-HT_{2A} et 5-HT_{1A} qui sont responsables respectivement de contraction et de relaxation musculaire (Cazzola and Matera, 2000). Toutefois, il a été démontré que la stimulation de récepteurs 5-HT_{1A} de cellules musculaires lisses respiratoires permettait de potentialiser une bronchoconstriction induite par l'activation de récepteurs 5-HT₂ (Germonpré et al. 1998).

Bien que la sérotonine seule soit capable d'induire la bronchoconstriction, il a été montré une interconnexion des système sérotoninergiques et cholinergiques. En effet, des récepteurs sérotoninergiques présynaptiques sont également capables de moduler (inhiber ou potentialiser) la libération de neurotransmetteurs provenant de fibres nerveuses cholinergiques et non-cholinergiques des voies respiratoires (Germonpré et al., 1998 ; Mendez-Enriquez 2021).

Outre le rôle de la sérotonine et de ses récepteurs dans le contrôle de la contractilité des fibres musculaires lisses bronchiques, le système sérotoninergique apparaît également important dans une autre composante importante des troubles obstructif : le remodelage des voies respiratoires. Les récepteurs sérotoninergiques notamment les récepteurs 5-HT2 jouent un rôle important dans le contrôle de la prolifération des cellules musculaires lisses bronchiques humaines et dans la libération du facteur pro-fibrotique TGF-β1 (Löfdhal et al. 2018).

Il est important de noter que certaines altérations structurelles des voies respiratoires sont des caractéristiques communes de plusieurs troubles respiratoires. En effet, une réactivité bronchique exacerbée est également observée dans d'autres pathologies pulmonaires (rhinite, bronchite chroniques, pathologies interstitielles pulmonaires, mucoviscidose ou encore dans la bronchopneumopathie chronique obstructive (BPCO)). La BPCO est une pathologie qui se caractérise par une obstruction des voies respiratoires généralement causée par une bronchite chronique, un emphysème ou les deux. Dans la BPCO, l'obstruction des voies respiratoires résulte d'une sécrétion chronique et excessive de mucus anormal, d'une inflammation, d'un bronchospasme et d'une infection. Il existe très peu de traitements actuellement disponibles pour soulager les symptômes de la BPCO, prévenir les exacerbations, préserver une fonction pulmonaire optimale et améliorer les activités quotidiennes et la qualité de vie des patients.

Le lien entre la sérotonine et la BPCO a clairement été établi. En effet, des taux de sérotonine circulants significativement plus élevés ont été observés chez des patients atteints de BPCO ce qui semble être lié à l'aggravation de l'obstruction des voies respiratoires et au taux de mortalité (Pirina et al. 2018 ; Meier et al. 2017).

Il apparait donc essentiel de développer de nouvelles approches pharmacologiques permettant de contrôler la contraction bronchique, notamment en réponse à la sérotonine, et gérer la réactivité exacerbée des voies respiratoires dans les maladies respiratoires, afin de limiter le plus possible l'évolution clinique des patients présentant des troubles bronchoconstrictifs tels que l'asthme ou la BPCO.

### Présentation de l'invention

La présente invention vise à proposer un tel traitement. La présente invention a pour objectif de palier les inconvénients précités en proposant un traitement efficace de pathologies respiratoires inflammatoires et notamment de pathologies respiratoires inflammatoires chroniques.

Il a été découvert par les présents inventeurs qu'un tel objectif est atteint par l'administration d'au moins une phytoecdysone et/ou au moins un dérivé hémi-synthétique de 20-hydroxyecdysone, cette administration induisant, de manière inattendue, une bronchodilatation et prévenant le bronchospasme chez les mammifères, dans le cadre d'une pathologie respiratoire inflammatoire. L'administration chronique d'au moins une phytoecdysone et/ou au moins un dérivé hémi-synthétique de 20-hydroxyecdysone induit une réduction de la réactivité bronchique exacerbée dans un modèle murin d'asthme sévère allergique. Cette diminution de la réactivité bronchique exacerbée est à la fois observée lors d'un test de bronchoconstriction induit par la méthacholine et de manière encore plus importante, lorsqu'il est induit par la sérotonine. De manière remarquable, cette diminution de la bronchoconstriction en réponse à la sérotonine est accompagnée de la réduction sélective de l'expression génique d'un récepteur du système sérotoninergique. En effet, à la suite d'une exposition chronique de phytoecdysone et/ou de dérivé hémi-synthétique de 20-hydroxyecdysone, l'expression génique du récepteur à la sérotonine 5-HT_{1A} est significativement réduite au niveau des bronches de mammifères. L'expression génique des autres récepteurs 5-HT testés (5-HT_{1B}, 5-HT_{2A}, 5-HT_{2B}, 5-HT₇) et du transporteur à la sérotonine (5-HTT) au niveau bronchique n'est pas significativement affectée par une administration chronique de phytoecdysone et/ou de dérivé hémi-synthétique de 20-hydroxyecdysone.

La réactivité bronchique exacerbée est associée à un remodelage pulmonaire massif. De manière tout à fait intéressante, l'administration chronique d'au moins une phytoecdysone et/ou au moins un dérivé hémi-synthétique de 20-hydroxyecdysone chez des mammifères asthmatiques diminue le remodelage des voies respiratoires des animaux. En effet, chez les souris asthmatiques traitées, l'inflammation péribronchique et périvasculaire sont diminuées. De plus, l'augmentation de la masse musculaire bronchique et le dysfonctionnement de la barrière épithéliale associés à l'asthme sévère semblent moins importants chez les animaux traités. Cette diminution de l'hyperplasie des cellules musculaires lisses bronchiques expliquerait la diminution de la réactivité bronchique exacerbée chez les souris asthmatiques traitées par l'administration chronique de phytoecdysone et/ou de dérivé hémi-synthétique de 20-hydroxyecdysone.

Ainsi, selon un premier aspect, la présente invention concerne une composition comprenant au moins une phytoecdysone et/ou au moins un dérivé hémi-synthétique de 20-hydroxyecdysone, pour son utilisation dans le traitement d'une pathologie respiratoire inflammatoire chez le mammifère, tel que défini dans les revendications.

On entend dans la présente description, par le terme « traitement », l'obtention d'un effet pharmacologique et physiologique souhaité. Le terme « traitement », tel qu'il est utilisé dans la présente description, comprend la prévention ou la prévention partielle d'un ou plusieurs des symptômes de la maladie et/ou la guérison partielle ou totale de la maladie et/ou la disparition totale ou partielle d'un ou plusieurs de ses symptômes.

On entend dans la présente description, par « au moins un », un seul composé (une phytoecdysone ou un dérivé hémi-synthétique de 20-hydroxyecdysone) ou un mélange de plusieurs tels composés.

Dans des modes particuliers de réalisation, l'invention répond en outre aux caractéristiques suivantes, mises en œuvre séparément ou en chacune de leurs combinaisons techniquement opérantes.

Une phytoecdysone utilisable selon l'invention est par exemple la 20-hydroxyecdysone.

On entend notamment par dérivés hémi-synthétiques de 20-hydroxyecdysone, les composés connus objet de la demande de brevet WO 2015/177469 dans laquelle leur obtention par hémisynthèse est décrite.

Les phytoecdysones et les dérivés hémi-synthétiques de 20-hydroxyecdysone sont avantageusement purifiés au grade pharmaceutique.

Selon un mode particulier de réalisation, la composition objet de la présente invention comporte de la 20-hydroxyecdysone et/ou au moins un dérivé hémi-synthétique de 20-hydroxyecdysone.

La 20-hydroxyecdysone utilisée est de préférence sous forme d'un extrait de végétaux riches en 20-hydroxyecdysone ou d'une composition comportant à titre d'agent actif la 20-hydroxyecdysone. Des extraits de végétaux riches en 20-hydroxyecdysone sont par exemple des extraits de *Stemmacantha carthamoides* (aussi appelée *Leuzea carthamoides*), *Cyanotis arachnoidea, Cyanotis vaga et Pfaffia paniculata*.

Les extraits obtenus sont de préférence purifiés au grade pharmaceutique.

Dans un mode de réalisation la 20-hydroxyecdysone est sous forme d'extrait de plante ou d'une partie de plante, ladite plante étant choisie parmi les végétaux contenant au moins 0,5% de 20-hydroxyecdysone en poids sec dudit végétal, ledit extrait comportant au moins 95%, et de préférence au moins 97%, de 20-hydroxyecdysone. Ledit extrait est de préférence purifié au grade pharmaceutique.

Ledit extrait est appelé par la suite BIO101. Il comporte de façon remarquable entre 0 et 0,05 %, en poids sec de l'extrait, d'impuretés, comme des composés mineurs, susceptibles d'affecter l'innocuité, la disponibilité ou l'efficacité d'une application pharmaceutique dudit extrait.

Selon un mode de réalisation de l'invention, les impuretés sont des composés à 19 ou 21 atomes de carbone, tels que la Rubrostérone, la Dihydrorubrostérone ou la Poststérone.

La plante à partir de laquelle est produit BIO101 est de préférence choisie parmi *Stemmacantha carthamoides* (aussi appelée *Leuzea carthamoides*), *Cyanotis arachnoidea*, *Cyanotis vaga et Pfaffia paniculata*.

Selon un mode de réalisation particulier, la pathologie respiratoire inflammatoire est une pathologie respiratoire inflammatoire chronique.

Selon un mode de réalisation préféré, la composition objet de la présente invention est utilisée en tant que bronchodilatateur dans le traitement de la pathologie respiratoire inflammatoire chez le mammifère.

La composition objet de la présente invention est destinée à être utilisée pour induire une bronchodilatation chez un patient atteint d'une pathologie respiratoire inflammatoire. Ainsi, dans des modes de réalisation particuliers, l'invention vise une composition comprenant au moins une phytoecdysone et/ou au moins un dérivé hémi-synthétique de 20-hydroxyecdysone, pour son utilisation dans l'induction d'une bronchodilatation dans le traitement d'un mammifère atteint d'une pathologie respiratoire inflammatoire.

Dans la présente description, le terme "bronchodilatateur" fait référence à tout composé qui dilate les bronches et les bronchioles et, ce faisant, augmente le débit d'air vers les poumons. Ils sont utiles dans les troubles bronchoconstrictifs tels que l'asthme, qui impliquent une obstruction du flux d'air et un bronchospasme.

Dans la présente description, le terme "bronchodilatation" fait référence à l'expansion des passages d'air bronchiques pour traiter ou prévenir un trouble bronchoconstrictif.

Dans la présente description, le terme "trouble bronchoconstrictif" fait référence à tout trouble ou maladie liés à la réduction du diamètre interne de la voie bronchique, par exemple une bronche ou des bronches, y compris, mais sans s'y limiter, l'asthme, la bronchopneumopathie chronique obstructive (BPCO) et la mucoviscidose.

Selon un mode de réalisation particulier, l'invention vise une composition comprenant au moins une phytoecdysone et/ou au moins un dérivé hémi-synthétique de 20-hydroxyecdysone, pour son utilisation dans le traitement de l'asthme, la bronchopneumopathie chronique obstructive ou la mucoviscidose.

La composition objet de la présente invention est en outre destinée à être utilisée en prévention ou réduction de la réactivité bronchique exacerbée chez un patient atteint pathologie respiratoire inflammatoire. Ainsi, dans des modes de réalisation particuliers, l'invention vise une composition comprenant au moins une phytoecdysone et/ou au moins un dérivé hémi-synthétique de 20-hydroxyecdysone, pour son utilisation dans la prévention ou la réduction d'une réactivité bronchique exacerbée dans le traitement d'un mammifère atteint d'une pathologie respiratoire inflammatoire.

Selon l'invention, la réactivité bronchique exacerbée désigne une anomalie des voies respiratoires qui consiste en une réponse exagérée de rétrécissement des voies respiratoires à de nombreux déclencheurs environnementaux, tels que les allergènes ou de l'exercice, mais sans s'y limiter. La réactivité bronchique exacerbée peut être une altération fonctionnelle du système respiratoire causée par une inflammation ou un remodelage des voies respiratoires. La réactivité bronchique exacerbée peut être causée par un dépôt de collagène, un bronchospasme, une hypertrophie du muscle lisse des voies respiratoires, une contraction du muscle lisse des voies respiratoires, une sécrétion de mucus, des dépôts cellulaires, une destruction épithéliale, une altération de la perméabilité épithéliale, une altération de la fonction ou de la sensibilité du muscle lisse, des anomalies du parenchyme pulmonaire et/ou des maladies infiltrantes dans et autour des voies respiratoires.

Beaucoup de ces facteurs causaux peuvent être associés à l'inflammation. La réactivité bronchique exacerbée qui est visée par le traitement avec la composition objet de la présente invention est associée à l'inflammation des voies respiratoires (par exemple, la production de cytokines inflammatoires).

Dans un mode de réalisation particulier, l'invention vise la composition comprenant au moins une phytoecdysone et/ou au moins un dérivé hémi-synthétique de 20-hydroxyecdysone pour son utilisation dans le traitement d'une altération de la fonction respiratoire liée à la voie sérotoninergique chez le mammifère atteint d'une pathologie respiratoire inflammatoire.

Dans un mode de réalisation particulier, l'invention vise la composition comprenant au moins une phytoecdysone et/ou au moins un dérivé hémi-synthétique de 20-hydroxyecdysone pour son utilisation dans le traitement d'une altération de la fonction respiratoire liée au récepteur 5-HT_{1A} de la voie sérotoninergique chez le mammifère atteint d'une pathologie respiratoire inflammatoire.

Dans un mode de réalisation particulier, les phytoecdysones sont administrées à une dose comprise entre 3 et 15 milligrammes par kilogramme par jour chez l'humain. On entend ici par phytoecdysone, aussi bien les phytoecdysones de manière générale, notamment la 20-hydroxyecdysone et préférentiellement sous forme d'extrait, que les dérivés hémi-synthétiques de 20-hydroxyecdysone.

De préférence, les phytoecdysones sont administrées à une dose de 200 à 1000 mg/jour, en une ou plusieurs prises, chez un humain adulte, et une dose de 5 à 350 mg/jour, en une ou plusieurs prises, chez l'humain enfant ou nourrisson. On entend ici par phytoecdysone, aussi bien les phytoecdysones de manière générale, notamment la 20-hydroxyecdysone et préférentiellement sous forme d'extrait, que les dérivés hémi-synthétiques de 20-hydroxyecdysone.

Selon la présente invention, ledit au moins un dérivé hémi-synthétique de 20-hydroxyecdysone est choisi parmi :
- un composé de formule générale (I) : 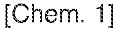 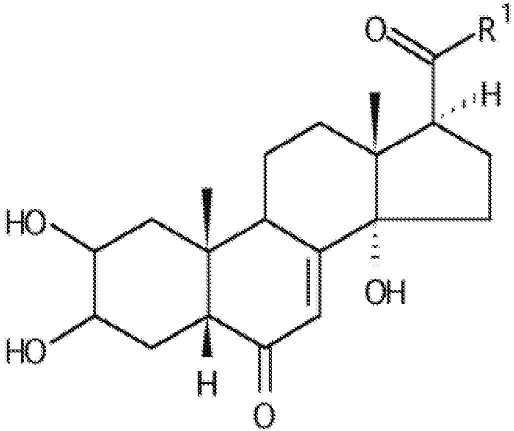 dans laquelle :
   ∘ **R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆) ; un groupement CH₂Br ;
      **W** étant un hétéroatome choisi parmi N, O et S, de préférence O et encore plus préférentiellement S ; et,
- un composé étant de formule (II) :

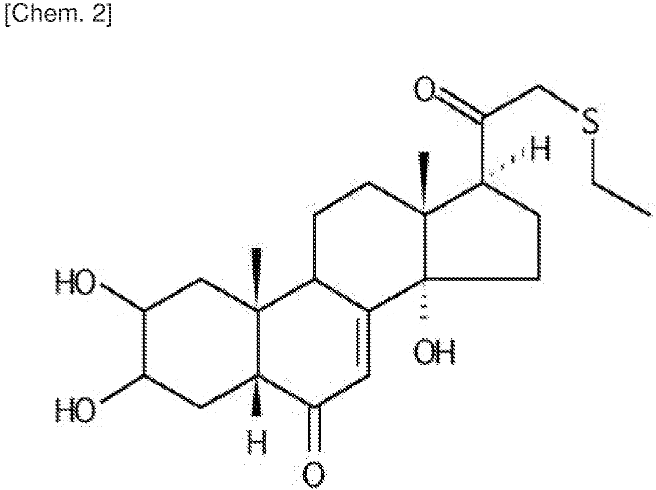

Dans le cadre de la présente invention on entend par « (C₁-C₆) », tout groupe alkyle de 1 à 6 atomes de carbones, linéaire ou ramifié, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe méthyle, éthyle, iso-propyle ou t-butyle, en particulier d'un groupe méthyle ou éthyle, plus particulièrement d'un groupe méthyle.

Dans le cadre de la présente invention on entend par hétérocycle de préférence un cycle comprenant 5 ou 6 atomes dont un ou deux hétéroatomes (O, S ou N), les atomes restants étant des atomes de carbone.

Dans un mode de réalisation préféré de la présente invention, dans la formule générale (1) :
- **R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆) ;
   **W** étant un hétéroatome choisi parmi N, O et S, de préférence O et de préférence encore S.

Dans des modes particuliers de réalisation de la présente invention ledit au moins un dérivé hémi-synthétique de 20-hydroxyecdysone est un composé choisi parmi les composés suivants :
- **n° 1 :** (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one,
- **n**° **2** : (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
- **n° 3** : (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
- **n° 4 :** (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
- **n° 5 :** (2S,3R,5R,10R,13R,14S,17S)-17-[2-(3-diméthylaminopropyl (méthyl)amino)acétyl]-2,3,14-trihydroxy-10,13-diméthyl-2,3,4,5,9,11,12,15,16,17-décahydro-1H-cyclopenta[a]phénanthrèn-6-one;
- **n° 6 :** 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
- **n**° **7** : (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
- **n° 8 :** (2S,3R,5R,10R,13R,14S,17S)-2,3,14-trihydroxy-17-[2-(2-hydroxyéthylsulfanyl)acétyl]-10,13-diméthyl-2,3,4,5,9,11,12,15,16,17-décahydro-1H cyclopenta[a]phénanthrèn-6-one.

Dans des modes de réalisation la composition objet de la présente invention est une composition pharmaceutique contenant, en tant que principe actif, au moins une phytoecdysone et/ou au moins un dérivé hémi-synthétique de 20-hydroxyecdysone, dans un véhicule pharmaceutiquement acceptable.

On entend dans la présente description, par « véhicule pharmaceutiquement acceptable », tout véhicule utile pour la préparation d'une composition pharmaceutique et qui est généralement sûr, non toxique et ni biologiquement ni autrement indésirable pour le sujet à traiter, en particulier pour les mammifères et notamment les humains.

Le véhicule de la composition pharmaceutique selon l'invention peut aussi bien être solide que semi-solide ou liquide. Il peut s'agir d'un diluant, d'un adjuvant ou de tout autre véhicule classique en lui-même pour la constitution des compositions pharmaceutiques.

La composition pharmaceutique selon l'invention peut se présenter sous toute forme galénique, notamment sous forme adaptée à une administration par voie parentérale, intranasale, rectale, pulmonaire, intrathécale, systémique ou topique. Préférentiellement, elle se présente sous une forme convenant à une administration par voie orale. A titre d'exemples de telles formes galéniques, non limitatives de l'invention, on peut citer les formes de granulés, poudre, comprimés, gélules, pilules, sirop, solution ou suspension buvable, etc.

L'administration du composé utilisé selon l'invention au sujet à traiter peut être réalisée par toute voie classique en elle-même, notamment par voie parentérale, par exemple par voie sous-cutanée, sous-durale, intraveineuse, intramusculaire, intrathécale, intrapéritonéale, intracérébrale, intra-artérielle ou intra-lésionnelle ; par voie intranasale ; par voie rectale ; par voie pulmonaire, par exemple par aérosol ou inhalation ; ou même par voie topique. Elle est préférentiellement réalisée par voie orale.

Tout sel conventionnel pharmaceutiquement acceptable du composé de formule générale (I), peut être mis en œuvre selon l'invention. A titre d'exemples, on peut citer les chlorures, bromures, formiates, acétates, etc.

On entend dans la présente description, par « sel pharmaceutiquement acceptable », de manière classique en elle-même, tout sel du composé de formule générale (I) comprenant, en tant que contre-ion, une substance qui ne produit aucune réaction adverse, allergique ou autrement indésirable lorsqu'elle est administrée à un sujet, en particulier à un mammifère.

La composition selon l'invention, dans sa forme souhaitée, peut être préparée par toute méthode classique en elle-même pour la préparation de compositions pharmaceutiques.

La composition selon l'invention peut contenir un ou plusieurs excipients / additifs classiques en eux-mêmes pour la constitution des compositions pharmaceutiques, par exemple choisis parmi les conservateurs, les agents édulcorants, aromatisants, de charge, désintégrants, mouillants, émulsifiants, tensioactifs, dispersants, lubrifiants, stabilisants, tampons, antibactériens, antifongiques, etc., ou l'un quelconque de leurs mélanges; et/ou tout composé permettant une libération rapide, prolongée ou retardée, et/ou ciblée, du principe actif après son administration au sujet.

La composition selon l'invention peut en outre contenir un ou plusieurs principes actifs autres que les phytoecdysones ou dérivés hémi-synthétiques de 20-hydroxyecdysone, ces principes actifs pouvant ou non agir de manière synergique avec lesdites phytoecdysones ou lesdits dérivés hémi-synthétiques de 20-hydroxyecdysone.

La composition pharmaceutique selon l'invention est de préférence formulée sous forme de doses unitaires.

L'invention s'exprime également dans les termes d'un procédé de traitement d'une pathologie respiratoire inflammatoire, comprenant l'administration à un sujet en ayant besoin d'une quantité thérapeutiquement efficace d'une composition comprenant au moins une phytoecdysone et/ou au moins un dérivé hémi-synthétique de 20-hydroxyecdysone. Ce procédé peut répondre à l'une ou plusieurs des caractéristiques décrites ci-avant en référence à l'utilisation thérapeutique de la composition comprenant au moins une phytoecdysone et/ou au moins un dérivé hémi-synthétique de 20-hydroxyecdysone en tant que médicament.

On entend dans la présente description par « un sujet en ayant besoin » un sujet atteint ou susceptible d'être atteint par une pathologie respiratoire inflammatoire. Ce sujet peut notamment être un mammifère, et en particulier un humain.

On entend dans la présente description par « quantité thérapeutiquement efficace » la quantité de la composition administrée qui, lorsqu'elle est administrée à un sujet pour traiter la maladie, est suffisante pour assurer un tel traitement de la maladie. La quantité thérapeutiquement efficace de la composition utilisée selon l'invention dépend de plusieurs facteurs, tels que la maladie et sa gravité, l'âge, le poids, etc., du sujet à traiter, le ou les composé(s) particulier(s) de la composition utilisé(s), la voie et la forme d'administration, etc. La quantité thérapeutiquement efficace de la composition utilisée selon l'invention sera déterminée par le médecin pour chaque cas individuel. La composition peut par exemple être administrée au sujet en ayant besoin une, deux ou trois fois par jour, sur une longue période, à intervalles réguliers, ou de manière ciblée.

L'invention s'exprime également dans les termes d'une utilisation d'une composition comprenant au moins une phytoecdysone et/ou au moins un dérivé hémi-synthétique de 20-hydroxyecdysone pour la fabrication d'un médicament pour le traitement d'une pathologie respiratoire inflammatoire. Cette utilisation peut contenir une ou plusieurs des caractéristiques présentées ci-avant, mises en œuvre séparément ou en chacune de leurs combinaisons techniquement opérantes.

### Brève description des figures

L'invention sera mieux comprise à la lecture de la description suivante, donnée à titre d'exemple nullement limitatif, et faite en se référant aux figures qui représentent :

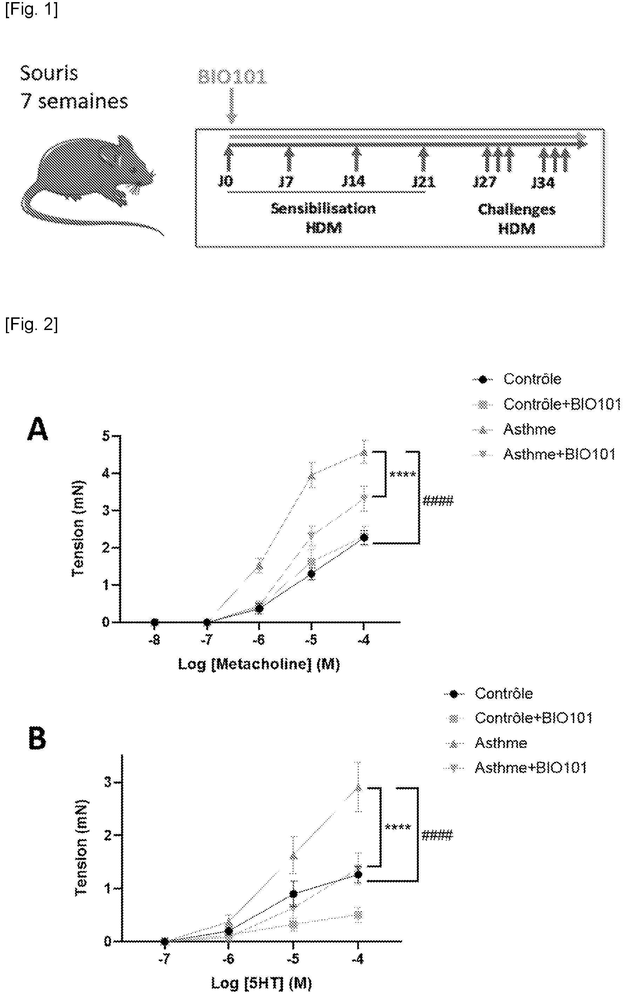

[Fig. 1] La figure 1 représente le chronogramme de l'étude HDM (signifiant *"House Dust Mite"* en terminologie anglo-saxonne) dans laquelle des souris modèle asthme allergique sont traitées avec BIO101 ;
[Fig. 2] La figure 2 illustre la courbe de réponse de contraction des anneaux bronchiques de souris saines (contrôle), ou asthmatiques (asthme) ainsi que les effets de la molécule BIO101 (50 mg/kg/jour) sur la contraction des anneaux bronchiques de souris contrôle (contrôle+BIO101) et asthmatiques (Asthme+BIO101). La bronchoconstriction a été induite par la stimulation de doses cumulatives de (**A**) méthacholine (10⁻⁸M à 10⁻⁴M) ou de **(B)** 5-HT (10⁻⁸M à 10⁻⁴M). Les valeurs sont présentées sous forme de moyennes ± SEM avec p<0,001^{####} contrôle vs Asthme, et Asthme vs Asthme+BIO101, p<0,001****. ;

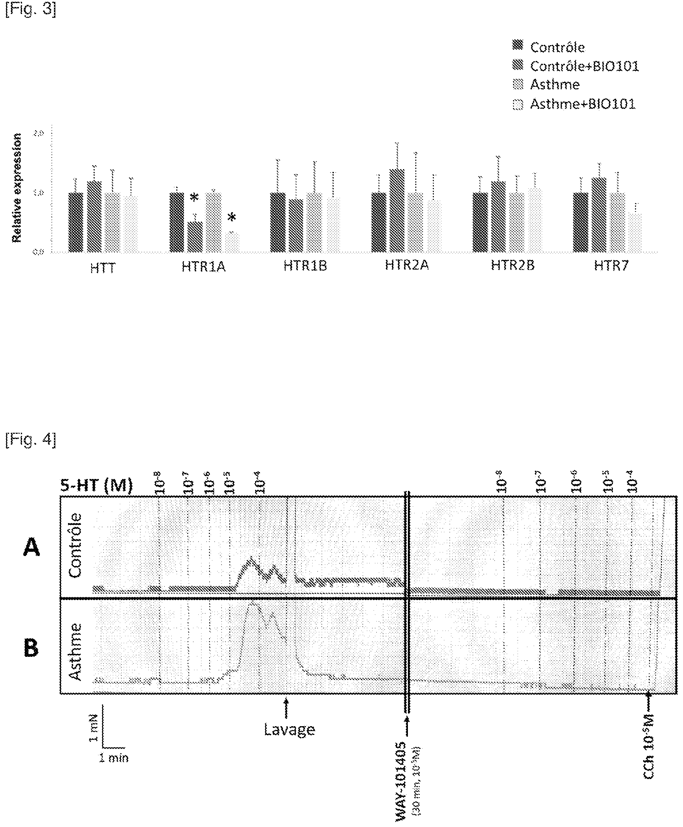

[Fig. 3] La figure 3 représente un histogramme illustrant l'expression génique des récepteurs à la 5-HT et de son transporteur au niveau des bronches de souris saines (contrôle), contrôles et traitées avec BIO101 (contrôle+BIO101), asthmatiques (asthme) et asthmatiques traitées avec BIO101 (asthme+BIO101). Les valeurs sont présentées sous forme de moyennes ± SEM. Les valeurs sont présentées sous forme de moyennes ± SEM avec p<0,05.
[Fig. 4] La figure 4 représente les tracés représentatifs des enregistrements en temps réel de la réponse d'anneaux bronchiques de souris saines (**A** : contrôle) et de souris modèles d'asthme allergique sévère (**B** : asthme) suite à une administration séquentielle de la sérotonine avant puis après incubation avec le WAY-101405, un antagoniste spécifique des récepteurs 5-HT1A. Barre d'échelle : 1 minute pour l'axe des abscisses et 1mN pour l'axe des ordonnées.

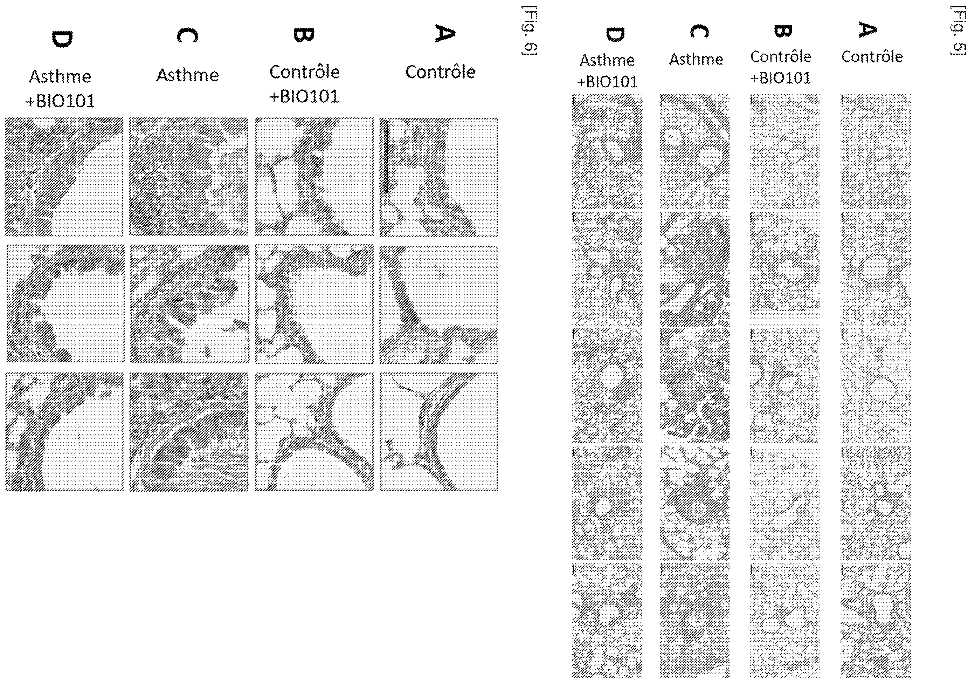

[Fig. 5] La Figure 5 représente des images représentatives de sections de poumons colorées à l'hématoxyline et Eosine de souris saines (**A** : contrôle) et traitées avec BIO101 (**B** : contrôle+BIO101), asthmatiques (**C** : asthme) et asthmatiques traitées avec BIO101 (D : asthme+BIO101). La barre d'échelle représente 100 µm.
[Fig. 6] La Figure 6 représente des images à fort grossissement, représentatives de sections de poumons colorées à l'hématoxyline et Eosine de souris saines (**A** : contrôle) et traitées avec BIO101 (**B** : contrôle+BIO101), asthmatiques (C : asthme) et asthmatiques traitées avec BIO101 (**D** : asthme+BIO101). La barre d'échelle représente 100 µm.

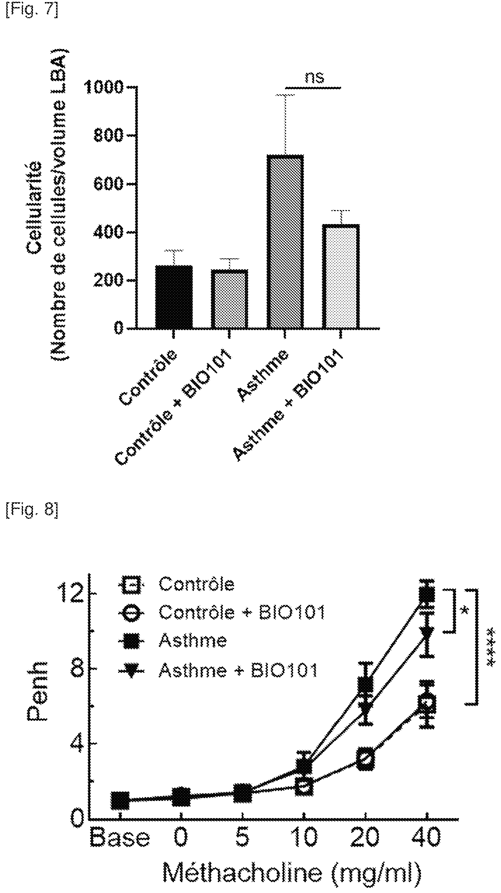

[Fig. 7] La Figure 7 représente les valeurs de la cellularité des liquides bronchoalvéolaires de souris contrôles (n=9), de souris contrôles traitées avec BIO101 (n=9), de souris asthmatiques (n=10) et de souris asthmatiques traitées avec BIO101 (n=11). La cellularité est calculée en divisant le nombre total de cellules de l'échantillon, par le volume de liquide bronchoalvéolaire récolté.
[Fig. 8] La Figure 8 représente la courbe de la valeur de la Penh de souris contrôles (n=10, carrés blancs), de souris contrôles traitées avec BIO101 (n=10 ; ronds blancs), de souris asthmatiques (n=10 ; carrés noirs) et de souris asthmatiques traitées avec BIO101 (n=11 ; triangles noirs), mesurée par pléthysmographie, en réponse à des doses croissantes de méthacholine avec *p<0,05 et ****p<0,0001.

### Description des modes de réalisation

Dans la présente description, n correspond à la taille de l'échantillon et p correspond à la « p-valeur » utilisée pour quantifier la significativité statistique d'un résultat.

Un test de Mann-Whitney a été effectué pour les comparaisons entre deux groupes. Un test *Two way* ANOVA a été utilisé pour les comparaisons multiples des études de contractilité bronchique. L'analyse des données a été réalisée à l'aide du logiciel GraphPad Prism 9 (GraphPad Software, Inc., La Jolla, CA, USA). L'invention sera décrite ci-après dans le contexte particulier d'un de ses domaines d'application préférés, non limitatif.

### 1. Description de l'étude :

Des souris âgées de 7 semaines sont sensibilisées par voie cutanée à des extraits d'acariens (HDM pour *House Dust Mite*) à J0, 7, 14 et 21. Après cette phase de sensibilisation cutanée à l'allergène, les souris reçoivent l'allergène par voie nasale (phase de challenge) à J27, 28, 29, 34, 35 et 36 pour induire une exacerbation bronchique. Ce modèle d'asthme allergique sévère a été caractérisé comme imitant la pathologie humaine (Dilasser et al. Thorax 2021).

BIO101 (lot RL11T1204A0) a été administré aux souris dans l'eau de boisson 7 jours sur 7, à une dose de 50mg/kg/jour. L'eau de boisson contenant BIO101 a été remplacée deux fois par semaine jusqu'au sacrifice des animaux.

### 2. Activité biologique de BIO101 dans un modèle murin d'asthme allergique sévère

### a. Analyse des effets du traitement BIO101 sur la bronchoconstriction

L'évaluation de l'effet de l'administration chronique du traitement par BIO101 sur la contraction *ex vivo* des anneaux bronchiques murins a été réalisé en chambre d'organe isolé (mesure de la tension isométrique, myographe Mulvany, DMT, Hinnerup, Danemark) (André-Grégoire et al., 2018).

Les bronches sont prélevées, nettoyées, coupées en anneaux et installées dans le myographe Mulvany. Les anneaux sont pré-tendus puis soumis à une stimulation avec du KCl à 60 mM, jusqu'à ce que l'amplitude des réponses contractiles soit stabilisée (2 ou 3 stimulations) avant l'initiation de chaque protocole expérimental.

A J38 après le début du protocole **(****Figure 1****),** les bronches de souris saines non-asthmatiques (contrôle ; n≥8), de souris saines non-asthmatiques traitées par BIO101 (contrôle+BIO101 ; n=8), de souris asthmatiques (Asthme ; n≥8) et de souris asthmatiques traitées par BIO101 (Asthme+BIO101 ; n≥8) ont été prélevées. La bronchoconstriction a été induite par la stimulation de doses cumulatives de méthacholine (10⁻⁷M à 10⁻⁴M) ou de sérotonine (10⁻⁷M à 10⁻⁴M).

Les expériences ont été réalisées sur 2 anneaux bronchiques de chaque souris. La molécule BIO101 a été ajoutée à une concentration de 10⁻⁵M dans les chambres d'organes isolées contenant les bronches des souris traitées. Ceci a permis de maintenir le traitement des tissus tout au long de l'expérience. Il est à noter que l'administration aigue de BIO101 dans la cuve contenant l'anneau bronchique ne modifie pas la réponse contractile à la méthacholine (0,41 ± 0,25mN) par rapport au contrôle interne sans BIO101 (0,37 ± 0,22mN ; p=ns) ni à la sérotonine (0,29 ± 0,14mN) par rapport au contrôle interne sans BIO101 (0,25 ± 0,12mN ; p=ns) (résultats antérieurs obtenus, non présentés ici).

De manière attendue, chez les souris asthmatiques, les bronchoconstrictions induites par la méthacholine et la sérotonine sont significativement plus élevées par rapport à celles des souris saine (p<0,0001 pour chacune des conditions), ce qui atteste que la sensibilisation aux acariens a induit une réactivité bronchique exacerbée **(****Figure 2****).**

Chez les souris asthmatiques, le traitement chronique par BIO101 diminue significativement la bronchoconstriction induite par la méthacholine chez les souris asthmatiques (Asthme *vs* Asthme+BIO101 ; p<0,0001). Néanmoins, le traitement ne prévient pas réactivité bronchique exacerbée des voies respiratoires et ne permet pas d'atteindre un niveau de bronchoconstriction comparable à celui de souris saines (Asthme+BIO101 vs Contrôle ; p=0,0025).

De la même manière, le traitement chronique par BIO101 diminue significativement la bronchoconstriction induite par la sérotonine chez les souris asthmatiques (Asthme vs Asthme+BIO101 ; p<0,0001). De manière remarquable, on observe que traitement chronique avec BIO101 induit une bronchoconstriction induite par la sérotonine comparable à celle observée avec des bronches de souris saines (Asthme+BIO101 vs Contrôle ; p=0,9997) démontrant que le traitement prévient complètement la réactivité bronchique exacerbée des voies respiratoires.

### b. Analyse de l'effet d'un traitement chronique par BIO101 sur l'expression génique des récepteurs et du transporteur de la 5-HT au niveau des bronches

A J38, les bronches de souris saines non-asthmatiques (contrôle ; n=10), de souris contrôles non-asthmatiques traitées par BIO101 (contrôles+BIO101 ; n=10), de souris asthmatiques (Asthme ; n=10) et de souris asthmatiques traitées par BIO101 (Asthme+BIO101 ; n=10) ont été prélevées. Après extraction de l'ARN de ces tissus (Trizol^{©}, ThermoFisher cat. 15596018), une analyse de l'expression des récepteurs de la sérotonine (5-HT1A, 5-HT1B, 5-HT2A, 5-HT2B et 5-HT7) et du transporteur de la sérotonine (5HTT) a été évaluée par PCR en temps réel (TaqMan^{™}, Universal PCR MasterMix, ThermoFisher cat. 4304437). La liste des références des tests commerciaux ThermoFisher Scientific utilisés pour la PCR en temps réel ayant permis d'obtenir les résultats pour chacun des gènes est donnée dans le tableau 1 ci-dessous :

**[Table 1]**

| **Symbole du gène** | **Référence Test** |
|---|---|
| Htt | Mm01213820_m1 |
| Htr7 | Mm00434133_m1 |
| Htr2a | Mm00555764_m1 |
| Htr2b | Mm00434123_m1 |
| Htr1a | Mm00434106_s1 |
| Htr1b | Mm00439377_s1 |
| | |
| Gapdh | Mm99999915_g1 |
| Hprt | Mm03024075_m1 |

Le traitement chronique des souris avec BIO101 induit une diminution significative et sélective de l'expression du gène du récepteur 5-HT1A dans les bronches **(****Figure 3****).** En effet, l'expression génique du récepteur 5-HT1A diminue de -49% ± 12% (p<0.05) chez des souris saines traitées avec BIO101 (contrôle+BIO101) et de manière encore plus importante chez les souris asthmatiques traitées avec BIO101 (asthme+BIO101) (-69 ± 3% ; p<0.05).

Le traitement avec BIO101 n'induit pas de modification des autres récepteurs ou du transporteur à la sérotonine, qu'il s'agisse de souris saines ou asthmatiques (5-HT1B, 5-HT2A, 5-HT2B et 5-HT7 et 5-HTT).

### c. Confirmation de l'implication du récepteur à la 5-HT1A à la bronchoconstriction induite par la sérotonine.

Des enregistrements en temps réel de la réponse des bronches de souris contrôles et asthmatiques suite à l'administration séquentielle de sérotonine à différentes concentrations (10⁻⁸M to 10⁻⁴M) ont été réalisés. Suite à cet enregistrement, les anneaux bronchiques ont été incubés pendant 30 minutes avec le WAY-101405, un antagoniste sélectif du récepteur 5-HT1A **(****Figure 4****).**

De manière attendue, la sérotonine induit une bronchoconstriction au niveau des anneaux bronchiques de souris saines (contrôles) et asthmatiques. La bronchoconstriction des bronches de souris asthmatiques **(****Figure 4**, **B)** étant plus importante que celle des souris saines **(****Figure 4****, A**). La bronchoconstriction induite par la sérotonine est totalement inhibée par l'antagoniste du récepteur 5-HT1A (WAY-101405) chez les souris témoins et asthmatiques **(****Figure 4****, A** et **B).**

Ce résultat suggère que la bronchoconstriction dépendante de la sérotonine est essentiellement médiée par l'activation du récepteur 5-HT1A.

L'ensemble de ces résultats, suggère fortement que la diminution sélective de l'expression des récepteurs 5-HT1A induite par un traitement chronique à BIO101 pourrait contribuer significativement à la prévention de la réactivité bronchique exacerbée des voies respiratoires dans un contexte pathologique inflammatoire.

### d. Analyse histopathologique des effets du traitement chronique par BIO101 au niveau des poumons

L'analyse histologique de sections de poumons colorés en hématoxyline et Eosine montre que la réactivité bronchique exacerbée des voies respiratoires de souris sensibilisées aux acariens (asthme, **Figure 5** en **C** et **Figure 6** en **C**) est associée à un remodelage pulmonaire massif caractérisé par une production de mucus, une forte infiltration cellulaire des voies respiratoires, une hypertrophie des cellules épithéliales, ainsi qu'une hyperplasie des cellules musculaires lisses, par rapport au poumon de souris saines (**Figure 5** en **A** et **Figure 6** en **A**)**.**

Chez des souris saines (contrôle), le traitement par BIO101 n'a aucun effet sur l'histologie pulmonaire ((**Figure 5** en **B** et **Figure 6** en **B**). En revanche, chez les souris asthmatiques, le traitement par BIO101 réduit l'inflammation péribronchique et périvasculaire ((**Figure 5** en **D** et **Figure 6** en **D**). De plus, l'augmentation de la masse musculaire bronchique et le dysfonctionnement de la barrière épithéliale associés à l'asthme sévère semblent moins importants chez les animaux ayant reçu un traitement chronique avec BIO101.

### e. Evaluation de la cellularité des liquides broncho-alvéolaires

A J38, du PBS stérile (entre 200 µl et 1 ml) a été administré par voie intratrachéale aux souris à l'aide d'un cathéter. Les cellules et le surnageant du liquide bronchoalvéolaire (LBA) sont récupérés et séparés par centrifugation. Le nombre total de cellules a été compté sur des lames de Kova par microscopie optique. La cellularité de chaque échantillon a été évaluée en effectuant le rapport du nombre total de cellules comptées, divisé par le volume de liquide bronchoalvéolaire. La cellularité représente l'infiltrat des cellules immunitaires (macrophages, lymphocyte, éosinophiles et neutrophiles).

Il n'y a pas de différence au niveau du contenu cellulaire des LBA de souris contrôles traitées avec BIO101 et de souris contrôles non traitées (respectivement 260±64 et 244±46, p=ns). De manière attendue, la cellularité des LBA de souris asthmatiques augmente fortement (721±248 ; +177,3%) par rapport au groupe contrôle **(****Figure 7****).** De manière intéressante, bien que non significatif, le traitement de souris asthmatiques avec BIO101 diminue l'inflammation présente dans les LBA (-39,8% ; p=ns).

Ce résultat est en adéquation avec l'analyse histologique des sections de poumons dans lequel on observe que le traitement avec BIO101 induit une diminution de l'inflammation et du remodelage des voies respiratoires.

### f. Analyse de la fonction respiratoire par pléthysmographie

Afin d'étudier si la réduction de l'inflammation au niveau des LBA pourrait induire une amélioration fonctionnelle après l'induction d'un asthme sévère, la fonction respiratoire a été évaluée par pléthysmographie. Cette technique permet de réaliser une étude longitudinale de la capacité respiratoire ainsi que du temps de pause expiratoire (Penh) de manière non invasive. Les changements de débit et de pression provoqués par la respiration sont mesurés avant et après l'exposition à une nébulisation de doses croissantes de NaCl puis de méthacholine. L'enregistrement total pour une souris dure 45 minutes. La souris vigile, non contrainte, est placée dans une chambre de pléthysmographie isolée et une seule dose de méthacholine est nébulisée à la souris pendant une minute, puis pendant 5 minutes où l'enregistrement des données s'effectue. Après ces 5 minutes, une nouvelle dose de méthacholine est nébulisée et ainsi de suite. Au cours des 45 minutes, la souris subit au total 5 nébulisations avec des doses croissantes de méthacholine diluées avec du NaCl (0, 5, 10, 20, 40 mg/ml).

Les variations de pressions mesurées par rapport à une chambre de référence permettent de définir différents paramètres respiratoires tels que les pics et les temps de pression inspiratoire et expiratoire ainsi qu'une grandeur sans unité appelée Penh (pour « *enhanced Pause* » en terminologie anglosaxonne) qui permet d'évaluer la broncho-constriction. En effet, la valeur de Penh, calculée à partir du signal de pression dans la chambre (Pb), est un index important à obtenir, car les variations de la valeur de Penh évoluent en parallèle avec celles de la résistance respiratoire et elle représente donc un paramètre prédictif des changements des propriétés résistives du système respiratoire (Hamelmann et al., 1997 ; Bergren, 2001 ; Onclinx et al., 2003). Les valeurs suivantes ont été calculées à partir de la Pb filtrée : la variation maximale de Pb pendant l'expiration (PEP pour « *Peak Expiratory Pressure »* en terminologie anglosaxonne), la modification maximale de Pb pendant l'inspiration (PIP pour « *Peak Inspiratory Pressure* » en terminologie anglosaxonne) et l'intervalle de temps (TR). Ensuite, la valeur Penh a été calculée comme suit : [Math. 1] $Penh = \left(PIP/PEP\right) \times Pause$ où [Math. 2] $Pause = \left(TE-TR\right) / TE$ TE étant le temps expiratoire (Adler et al., 2004).

L'augmentation de la résistance pulmonaire induite par la méthacholine en aérosol (Penh) des souris contrôles et des souris contrôles traitées avec BIO101 est comparable. Dans cette étude, BIO101 ne semble pas avoir d'effet sur la fonction respiratoire d'animaux sains **(****Figure 8****).**

De manière attendue et décrit dans la littérature (Dijoux et al., 2023), on observe que la valeur de Penh augmente significativement dans le groupe de souris asthmatiques (11,97±0,7) par rapport aux souris saines, contrôles (6,10±1,21 ; p<0,0001), à 40mg/ml de méthacholine.

Le traitement par BIO101 diminue significativement la *bronchoconstriction* exacerbée induite par la méthacholine. En effet, l'administration de BIO101 permet de diminuer la valeur de Penh de 18,1% (11,97±0,7 dans le groupe de souris asthmatiques *versus* 9,81±1,1 dans le groupe de souris asthmatiques traitées avec BIO101 ; p<0,05).

### Conclusion

Ces résultats démontrent l'intérêt de l'utilisation du traitement BIO101, dans le cadre de pathologie respiratoires inflammatoires afin de diminuer la réactivité bronchique exacerbée. En effet, BIO101 montre des effets bénéfiques important dans un modèle murin d'asthme allergique sévère, en particulier au niveau de la bronchoconstriction induite par la méthacholine et la sérotonine. L'administration chronique de BIO101 induit une diminution significative du récepteur 5-HT1A au niveau des bronches ainsi qu'une diminution de l'inflammation et du remodelage des voies respiratoires. Le *traitement* par BIO101 (50 mg/kg par jour dans l'eau de boisson) améliore la fonction respiratoire dans un modèle murin d'asthme allergique sévère. Cette amélioration de la fonction respiratoire peut être liée à la diminution de la cellularité retrouvée dans les LBA de souris ayant reçu BIO101. En effet, la diminution de l'infiltrat inflammatoire est un paramètre clef dans l'amélioration clinique des patients souffrant de pathologies respiratoires inflammatoires, telles que l'asthme.

### Références bibliographiques

Adler A, Cieslewicz G, Irvin CG. Unrestrained plethysmography is an unreliable measure of airway responsiveness in BALB/c and C57BL/6 mice. J. Appl. Physiol. (1985). (2004); 97(1 ):286-92.
André-Grégoire G, Dilasser F, Chesné J, Braza F, Magnan A, Loirand G, Sauzeau V. Targeting of Rac1 prevents bronchoconstriction and airway hyperresponsiveness. J Allergy Clin Immunol. 2018 Sep;142(3):824-833.e3. doi: 10.1016/j.jaci.2017.09.049. Epub 2017 Nov 16. PMID: 29155102.
Bergren DR. Chronic tobacco smoke exposure increases airway sensitivity to capsaicin in awake guinea pigs. j. Appl. Physiol. (2001); 90:695-704.
Bloom CI, Saglani S, Feary J, Jarvis D, Quint JK. Changing prevalence of current asthma and inhaled corticosteroid treatment in the UK: population-based cohort 2006-2016. Eur Respir J 2019 Apr;53(4).
Boehme SA, Lio FM, Sikora L, Pandit TS, Lavrador K, Rao SP, Sriramarao P. Cutting edge: serotonin is a chemotactic factor for eosinophils and functions additively with eotaxin. J Immunol. 2004 Sep 15;173(6):3599-603. doi: 10.4049/jimmunol.173.6.3599. PMID: 15356103.
Bossé Y. Asthmatic airway hyperresponsiveness: the ants in the tree. Trends Mol Med. 2012 Nov;18(11):627-33. doi: 10.1016/j.molmed.2012.09.002. Epub 2012 Oct 10. PMID: 23062358.
Boulet LP. Airway remodeling in asthma: update on mechanisms and therapeutic approaches. Curr Opin Pulm Med. 2018 Jan;24(1):56-62. doi: 10.1097/MCP.0000000000000441. PMID: 29076828.
Castro M, Corren J, Pavord ID, Maspero J, Wenzel S, Rabe KF, Busse WW, Ford L, Sher L, FitzGerald JM, Katelaris C, Tohda Y, Zhang B, Staudinger H, Pirozzi G, Amin N, Ruddy M, Akinlade B, Khan A, Chao J, Martincova R, Graham NMH, Hamilton JD, Swanson BN, Stahl N, Yancopoulos GD, Teper A. Dupilumab Efficacy and Safety in Moderate-to-Severe Uncontrolled Asthma. N Engl J Med. 2018 Jun 28;378(26):2486-2496. doi: 10.1056/NEJMoa1804092. Epub 2018 May 21. PMID: 29782217.
Cazzola I, Matera MG. 5-HT modifiers as a potential treatment of asthma. Trends Pharmacol Sci. 2000 Jan;21(1):13-6. doi: 10.1016/s0165-6147(99)01408-x. PMID: 10637649.
Chanez P, Wenzel SE, Anderson GP, Anto JM, Bel EH, Boulet LP, Brightling CE, Busse WW, Castro M, Dahlen B, Dahlen SE, Fabbri LM, Holgate ST, Humbert M, Gaga M, Joos GF, Levy B, Rabe KF, Sterk PJ, Wilson SJ, Vachier I. Severe asthma in adults: what are the important questions? J Allergy Clin Immunol. 2007 Jun;119(6):1337-48. doi: 10.1016/j.jaci.2006.11.702. Epub 2007 Apr 9. PMID: 17416409.
De Bie JJ, Henricks PA, Cruikshank WW, Hofman G, Jonker EH, Nijkamp FP, Van Oosterhout AJ. Modulation of airway hyperresponsiveness and eosinophilia by selective histamine and 5-HT receptor antagonists in a mouse model of allergic asthma. Br J Pharmacol. 1998 Jul;124(5):857-64. doi: 10.1038/sj.bjp.0701901. PMID: 9692769; PMCID: PMC1565459.
Delaney C, Gien J, Grover TR, Roe G, Abman SH. Pulmonary vascular effects of serotonin and selective serotonin reuptake inhibitors in the late-gestation ovine fetus. Am J Physiol Lung Cell Mol Physiol. 2011 Dec;301(6):L937-44. doi: 10.1152/ajplung.00198.2011. Epub 2011 Sep 9. PMID: 21908589; PMCID: PMC3233823.
Dijoux E, Klein M, Misme-Aucouturier B, Cheminant MA, de Carvalho M, Collin L, Hassoun D, Delage E, Gourdel M, Loirand G, Sauzeau V, Magnan A, Bouchaud G. Allergic Sensitization Driving Immune Phenotyping and Disease Severity in a Mouse Model of Asthma. Allergy Asthma Immunol Res. 2023 Mar;15(2):246-261.
Dilasser F, Rose L, Hassoun D, Klein M, Rousselle M, Brosseau C, Guignabert C, Taillé C, Dombret MC, Di Candia L, Heddebaut N, Bouchaud G, Pretolani M, Magnan A, Loirand G, Sauzeau V. Essential role of srnooth muscle Rac1 in severe asthma-associated airway remodelling. Thorax. 2021 Apr;76(4):326-334. doi: 10.1136/thoraxjnl-2020-216271. Epub 2021 Feb 4. PMID: 33542087; PMCID: PMC7982925.
Filip M, Bader M. Overview on 5-HT receptors and their role in physiology and pathology of the central nervous system. Pharmacol Rep. 2009 Sep-Oct;61(5):761-77. doi: 10.1016/s1734-1140(09)70132-x. PMID: 19903999.
Germonpré PR, Joos GF, Pauwels RA. Modulation by 5-HT1A receptors of the 5-HT2 receptor-mediated tachykinin-induced contraction of the rat trachea in vitro. Br J Pharmacol. 1998 Apr;123(8):1571-8. doi: 10.1038/sj.bjp.0701771. PMID: 9605563; PMCID: PMC1565327.
Global burden of 369 diseases and injuries in 204 countries and territories, 1990-2019: a systematic analysis for the Global Burden of Disease Study 2019. Lancet. 2020;396(10258):1204-22.
Goldsmith AM, Hershenson MB, Wolbert MP, Bentley JK. Regulation of airway smooth muscle alpha-actin expression by glucocorticoids. Am J Physiol Lung Cell Mol Physiol. 2007 Jan;292(1):L99-L106. doi: 10.1 152/ajplung.00269.2006. Epub 2006 Sep 15. PMID: 16980374.
Goto K, Chiba Y, Sakai H, Misawa M. Glucocorticoids inhibited airway hyperresponsiveness through downregulation of CPI-17 in bronchial smooth muscle. Eur J Pharmacol. 2008 Sep 4;591(1-3):231-6. doi: 10.1016/j.ejphar.2008.06.021. Epub 2008 Jun 11. PMID: 18577381.
Habib N, Pasha MA, Tang DD. Current Understanding of Asthma Pathogenesis and Biomarkers. Cells. 2022 Sep 5;11(17):2764. doi: 10.3390/cells11172764. MID: 36078171; PMCID: PMG9454904.
Hamelmann E, Schwarze J, Takeda K, Oshiba A, Larsen GL, Irvin CG, Gelfand EW. Noninvasive measurement of airway responsiveness in allergic mice using barometric plethysmography. Am. J. Respir. Crit. Care Med. (1997); 156:766-75.
Holgate ST, Chuchalin AG, Hébert J, Lötvall J, Persson GB, Chung KF, Bousquet J, Kerstjens HA, Fox H, Thirlwell J, Cioppa GD; Omalizumab 011 International Study Group. Efficacy and safety of a recombinant anti-immunoglobulin E antibody (omalizumab) in severe allergic asthma. Clin Exp Allergy. 2004 Apr;34(4):632-8. doi: 10.1111/j.1365-2222.2004.1916.x. PMID: 15080818.
Kitson SL. 5-hydroxytryptamine (5-HT) receptor ligands. Curr Pharm Des. 007;13(25):2621-37. doi: 10.2174/138161207781663000. PMID: 17897004.
Kushnir-Sukhov NM, Gilfillan AM, Coleman JW, Brown JM, Bruening S, Toth M, Metcalfe DD. 5-hydroxytryptamine induces mast cell adhesion and migration. J Immunol. 2006 Nov 1;177(9):6422-32. doi: 10.4049/jimmunol.177.9.6422. PMID: 17056574.
Lechin F, van der Dijs B, Lechin A, Orozco B, Lechin M, Báez S, Rada I, León G, Acosta E. Plasma neurotransmitters and cortisol in chronic illness: role of stress. J Med. 1994;25(3-4):181-92. PMID: 7996062.
Lechin F, van der Dijs B, Orozco B, Lechin M, Lechin AE. Increased levels of free serotonin in plasma of symptomatic asthmatic patients. Ann Allergy Asthma Immunol. 1996 Sep;77(3):245-53. doi: 10.1016/S1081-1206(10)63263-2. PMID: 8814052.
Lima C, Souza VM, Soares AL, Macedo MS, Tavares-de-Lima W, Vargaftig BB. Interference of methysergide, a specific 5-hydroxytryptamine receptor antagonist, with airway chronic allergic inflammation and remodelling in a murine model of asthma. Clin Exp Allergy. 2007 May;37(5):723-34. doi: 10.1111/j.1365-2222.2007.02700.x. PMID: 17456220.
Meier MA, Ottiger M, Vögeli A, Steuer C, Bernasconi L, Thomann R, Christ-Crain M, Henzen C, Hoess C, Zimmerli W, Huber A, Mueller B, Schuetz P. Activation of the Serotonin Pathway is Associated with Poor Outcome in COPD Exacerbation: Results of a Long-Term Cohort Study. Lung. 2017 Jun;195(3):303-311. doi: 10.1007/s00408-017-0004-7. Epub 2017 Apr 22. PMID: 28434116.
Ménard G, Turmel V, Bissonnette EY. Serotonin modulates the cytokine network in the lung: involvement of prostaglandin E2. Clin Exp Immunol. 2007 Nov;150(2):340-8. doi: 10.11 11/j.1365-2249.2007.03492.x. Epub 2007 Sep 5. PMID: 17822443; PMCID: PMC2219342.
Mims JW. Asthma: definitions and pathophysiology. Int Forum Allergy Rhinol. 2015 Sep;5 Suppl 1:S2-6. doi: 10.1002/alr.21609. PMID: 26335832.
Müller T, Dürk T, Blumenthal B, Grimm M, Cicko S, Panther E, Sorichter S, Herouy Y, Di Virgilio F, Ferrari D, Norgauer J, Idzko M. 5-hydroxytryptamine modulates migration, cytokine and chemokine release and T-cell priming capacity of dendritic cells in vitro and in vivo. PLoS One. 2009 Jul 31;4(7):e6453. doi: 10.1371/journal.pone.0006453. PMID: 19649285; PMCID: PMC2714071.
Nichols DE, Nichols CD. Serotonin receptors. Chem Rev. 2008 May;108(5):1614-41. doi: 10.1021/cr078224o. Epub 2008 May 14. PMID: 18476671.
Onclinx C. Relation entre la résistance pulmonaire totale et la Penh en fonction de la localisation anatomique de l'obstruction des voies aériennes (mémoire-diplôme d'étude approfondie). Université de Liège, Faculté de médecine vétérinaire : Liège, (2003), 24p.
Ortega HG, Liu MC, Pavord ID, Brusselle GG, FitzGerald JM, Chetta A, Humbert M, Katz LE, Keene ON, Yancey SW, Chanez P; MENSA Investigators. Mepolizumab treatment in patients with severe eosinophilic asthma. N Engl J Med. 2014 Sep 25;371(13):1198-207. doi: 10.1056/NEJMoa1403290. Epub 2014 Sep 8. Erratum in: N Engl J Med. 2015 Apr 30;372(18):1777. PMID: 25199059.
Pakala R, Willerson JT, Benedict CR. Effect of serotonin, thromboxane A2, and specific receptor antagonists on vascular smooth muscle cell proliferation. Circulation. 1997 Oct 7;96(7):2280-6. doi: 10.1161/01.cir.96.7.2280. PMID: 9337201.
Pirina P, Zinellu E, Paliogiannis P, Fois AG, Marras V, Sotgia S, Carru C, Zinellu A. Circulating serotonin levels in COPD patients: a pilot study. BMC Pulm Med. 2018 Nov 8;18(1):167. doi: 10.1186/s12890-018-0730-5. PMID: 30409142; PMCID: PMC6225723.
Reddel HK, Bateman ED, Becker A, Boulet LP, Cruz AA, Drazen JM, Haatltela T, Hurd SS, Inoue H, de Jongste JC, Lemanske RF, Jr., Levy ML, O'Byrne PM, Paggiaro P, Pedersen SE, Pizzichini E, Soto-Quiroz M, Szefler SJ, Wong GW, FitzGerald JM. A summary of the new GINA strategy: a roadmap to asthma control. Eur Respir J 2015; 46: 622-639.
Thomson NC, Bicknell S, Chaudhuri R. Bronchial thermoplasty for severe asthma. Curr Opin Allergy Clin Immunol. 2012 Jun;12(3):241-8. doi: 10.1097/ACI.0b013e32835335ca. PMID: 22475996.

## Revendications

1. Composition comprenant au moins une phytoecdysone et/ou au moins un dérivé hémi-synthétique de 20-hydroxyecdysone, pour son utilisation dans l'induction d'une bronchodilatation et/ou dans la prévention ou la réduction de la réactivité bronchique exacerbée, chez le mammifère atteint
d'une pathologie respiratoire inflammatoire, ledit dérivé hémi-synthétique de 20-hydroxyecdysone étant choisi parmi :
- un composé de formule générale (I) : 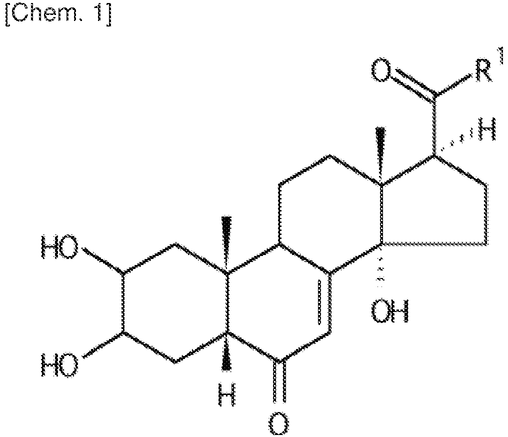 dans laquelle :
∘ **R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆) ; un groupement CH₂Br ;
**W** étant un hétéroatome choisi parmi N, O et S, de préférence O et encore plus préférentiellement S ; et,
- un composé étant de formule (II) :

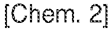

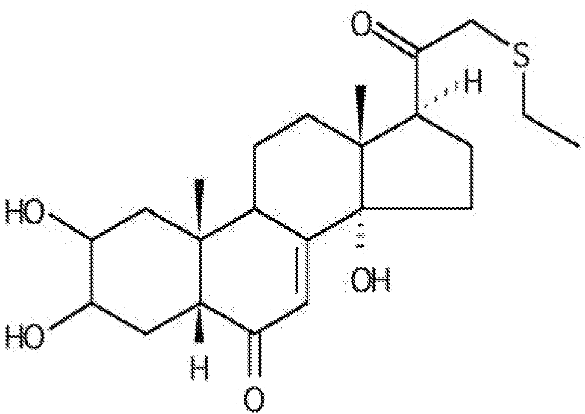

2. Composition pour son utilisation selon la revendication 1, dans laquelle la pathologie respiratoire inflammatoire est une pathologie respiratoire inflammatoire chronique.

3. Composition pour son utilisation selon l'une quelconque des revendications 1 à 2, dans laquelle pathologie respiratoire inflammatoire est l'asthme, la bronchopneumopathie chronique obstructive ou la mucoviscidose.

4. Composition pour son utilisation selon l'une quelconque des revendications 1 à 3, dans laquelle la composition est utilisée dans le traitement d'une altération de la fonction respiratoire liée à la voie sérotoninergique chez le mammifère atteint d'une pathologie respiratoire inflammatoire.

5. Composition pour son utilisation selon la revendication 4, dans laquelle la composition est utilisée dans le traitement d'une altération de la fonction respiratoire liée au récepteur 5-HT_{1A} de la voie sérotoninergique chez le mammifère atteint d'une pathologie respiratoire inflammatoire.

6. Composition pour son utilisation selon l'une quelconque des revendications 1 à 5, comportant de la 20-hydroxyecdysone.

7. Composition pour son utilisation selon la revendication 6, dans laquelle la 20-hydroxyecdysone est sous forme d'extrait de plante ou d'une partie de plante, ladite plante étant choisie parmi les végétaux contenant au moins 0,5% de 20-hydroxyecdysone en poids sec dudit végétal, ledit extrait comportant au moins 95%, et de préférence au moins 97%, de 20-hydroxyecdysone.

8. Composition pour son utilisation selon la revendication 7, comportant de façon remarquable entre 0 et 0,05 %, en poids sec de l'extrait, d'impuretés susceptibles d'affecter l'innocuité, la disponibilité ou l'efficacité d'une application pharmaceutique dudit extrait.

9. Composition pour son utilisation selon l'une quelconque des revendications 7 à 8, dans laquelle la plante est choisie parmi *Sternmacantha carthamoides, Cyanotis arachnoidea, Cyanotis vaga et Pfaffia paniculata*.

10. Composition pour son utilisation selon l'une quelconque des revendications 1 à 9, dans laquelle dans la formule générale (I) :
**R¹** est choisi parmi : un groupement (C₁-C₆)**W**(C₁-C₆) ; un groupement (C₁-C₆)**W**(C₁-C₆)**W**(C₁-C₆) ; un groupement (C₁-C₆)**W**(C₁-C₆)CO₂(C₁-C₆); un groupement (C₁-C₆)**A**, **A** représentant un hétérocycle éventuellement substitué par un groupement de type OH, OMe, (C₁-C₆), N(C₁-C₆), CO₂(C₁-C₆) ;
**W** étant un hétéroatome choisi parmi N, O et S, de préférence O et de préférence encore S.

11. Composition pour son utilisation selon l'une quelconque des revendications 1 à 10, dans laquelle ledit composé de formule générale (I) est choisi parmi :
- **n**°**1** : (2S,3R,5R,10R,13R,14S,17S)-2,3,14-trihydroxy-10,13-diméthyl-17-(2-morpholinoacétyl)-2,3,4,5,9,11,12,15,16,17-décahydro-1H-cyclopenta[a]phénanthrèn-6-one;
- **n°2** : (2S,3R,5R,10R,13R,14S,17S)-2,3,14-trihydroxy-17-[2-(3-hydroxypyrrolidin-1-yl)acétyl]-10,13-diméthyl-2,3,4,5,9,11,12,15,16,17-décahydro-1H-cyclopenta[a]phénanthrèn-6-one;
- **n**°**3** : (2S,3R,5R,10R,13R,14S,17S)-2,3,14-trihydroxy-17-[2-(4-hydroxy-1-pipéridyl)acétyl]-10,13-diméthyl-2,3,4,5,9,11,12,15,16,17-décahydro-1H-cyclopenta[a]phénanthrèn-6-one;
- **n**°**4** : (2S,3R,5R,10R,13R,14S,17S)-2,3,14-trihydroxy-17-[2-[4-(2-hydroxyéthyl)-1-pipéridyl]acétyl]-10,13-diméthyl-2,3,4,5,9,11,12,15,16,17-décahydro-1H-cyclopenta[a]phénanthrèn-6-one;
- **n°5 :** (2S,3R,5R,10R,13R,14S,17S)-17-[2-(3-diméthylaminopropyl(méthyl)amino)acétyl]-2,3,14-trihydroxy-10,13-diméthyl-2,3,4,5,9,11,12,15,16,17-décahydro-1H-cyclopenta[a]phénanthrèn-6-one;
- **n**°**6** : 2-[2-oxo-2-[(2S,3R,5R,10R,13R,14S,17S)-2,3,14-trihydroxy-10,13-diméthyl-6-oxo-2,3,4,5,9,11,12,15,16,17-décahydro-1H-cyclopenta[a]phénanthrèn-17-yl]éthyl]sulfanylacétate d'éthyle;
- **n°7** : (2S,3R,5R,10R,13R,14S,17S)-17-(2-éthylsulfanylacétyl)-2,3,14-trihydroxy-10,13-diméthyl-2,3,4,5,9,11,12,15,16,17-décahydro-1H-cyclopenta[a]phénanthrèn-6-one;
- **n**°**8** : (2S,3R,5R,10R,13R,14S,17S)-2,3,14-trihydroxy-17-[2-(2-hydroxyéthylsulfanyl)acétyl]-10,13-diméthyl-2,3,4,5,9,11,12,15,16,17-décahydro-1H-cyclopenta[a]phénanthrèn-6-one.

## Patentansprüche

1. Zusammensetzung, umfassend mindestens ein Phytoecdysteroid und/oder mindestens ein halbsynthetisches Derivat von 20-Hydroxyecdyson, zur Verwendung bei der Induktion einer Bronchodilatation und/oder zur Vorbeugung oder Verringerung einer verstärkten bronchialen Reaktivität bei einem Säugetier mit einer entzündlichen Atemwegserkrankung, wobei das halbsynthetische Derivat von 20-Hydroxyecdyson ausgewählt ist aus:
- einer Verbindung mit der allgemeinen Formel (I): 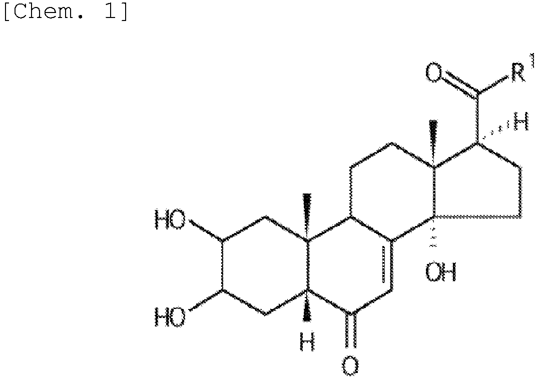 wobei:
∘ **R¹** ausgewählt ist aus: einer (C₁-C₆)**W**(C₁-C₆)-Gruppe; einer (C₁-C₆)**W**(C₁-C₆)**W**(C₁-C₆)-Gruppe; einer (C₁-C₆)**W**(C₁-C₆)CO₂(C₁-C₆)-Gruppe; einer (C₁-C₆)**A**-Gruppe, wobei **A** für einen Heterozyklus steht, der optional durch eine Gruppe vom Typ OH, OMe, (C₁-C₆), N(C₁-C₆), CO₂(C₁-C₆) substituiert ist; einer CH₂Br-Gruppe;
wobei **W** ein Heteroatom ist, das aus N, O und S, vorzugsweise O und noch bevorzugter S, ausgewählt ist; und
- einer Verbindung mit der Formel (II):

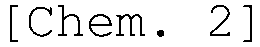

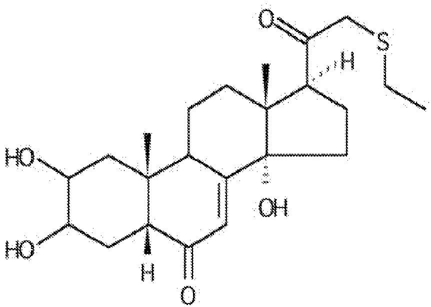

2. Zusammensetzung zur Verwendung gemäß Anspruch 1, wobei die entzündliche Atemwegserkrankung eine chronisch entzündliche Atemwegserkrankung ist.

3. Zusammensetzung zur Verwendung gemäß einem der Ansprüche 1 bis 2, wobei die entzündliche Atemwegserkrankung Asthma, chronisch obstruktive Lungenerkrankung oder Mukoviszidose ist.

4. Zusammensetzung zur Verwendung gemäß einem der Ansprüche 1 bis 3, wobei die Zusammensetzung zur Behandlung einer mit der serotoninergen Bahn verbundenen Beeinträchtigung der Atemfunktion bei einem Säugetier mit einer entzündlichen Atemwegserkrankung verwendet wird.

5. Zusammensetzung zur Verwendung gemäß Anspruch 4, wobei die Zusammensetzung zur Behandlung einer mit der serotoninergen 5-HT_{1A}-Rezeptor-Bahn verbundenen Beeinträchtigung der Atemfunktion bei einem Säugetier mit entzündlicher Atemwegserkrankung verwendet wird.

6. Zusammensetzung zur Verwendung gemäß einem der Ansprüche 1 bis 5, die 20-Hydroxyecdyson umfasst.

7. Zusammensetzung zur Verwendung gemäß Anspruch 6, wobei 20-Hydroxyecdyson in Form eines Extrakts von einer Pflanze oder einem Pflanzenteil vorliegt, wobei die Pflanze aus Pflanzen ausgewählt ist, die mindestens 0,5 % 20-Hydroxyecdyson, bezogen auf das Trockengewicht der Pflanze, enthalten, wobei der Extrakt mindestens 95 % und vorzugsweise mindestens 97 % 20-Hydroxyecdyson umfasst.

8. Zusammensetzung zur Verwendung gemäß Anspruch 7, die bemerkenswerterweise zwischen 0 und 0,05 % Verunreinigungen, bezogen auf das Trockengewicht des Extrakts, enthält, die die Sicherheit, Verfügbarkeit oder Wirksamkeit einer pharmazeutischen Anwendung des Extrakts beeinträchtigen können.

9. Zusammensetzung zur Verwendung gemäß einem der Ansprüche 7 bis 8, wobei die Pflanze aus *Stemmacantha carthamoides, Cyanotis arachnoidea, Cyanotis vaga* und *Pfaffia paniculata* ausgewählt ist.

10. Zusammensetzung zur Verwendung gemäß einem der Ansprüche 1 bis 9, wobei in der allgemeinen Formel (I): **R¹** ausgewählt ist aus: einer (C₁-C₆)W(C₁-C₆)-Gruppe; einer (C₁-C₆)**W**(C₁-C₆) **W**(C₁-C₆) -Gruppe; einer (C₁-C₆)W(C₁-C₆)CO₂(C₁-C₆)-Gruppe; einer (C₁-C₆) A-Gruppe, wobei A für einen Heterozyklus steht, der optional durch eine Gruppe vom Typ OH, OMe, (C₁-C₆), N(C₁-C₆), CO₂(C₁-C₆) substituiert ist;
wobei W ein Heteroatom ist, das aus N, O und S, vorzugsweise O und bevorzugter S, ausgewählt ist.

11. Zusammensetzung zur Verwendung gemäß einem der Ansprüche 1 bis 10, wobei die Verbindung der allgemeinen Formel (I) ausgewählt ist aus:
- **Nr. 1:** (2S,3R,SR,10R,13R,14S,17S)-2,3,14-Trihydroxy-10,13-dimethyl-17-(2-morpholinoacetyl)-2,3,4,5,9,11,12,15,16,17-decahydro-1H-cyclopenta[a]phenanthren-6-on;
- **Nr. 2:** (2S,3R,5R,10R,13R,14S,17S)-2,3,14-Trihydroxy-17-[2-(3-hydroxypyrrolidin-1-yl)acetyl]-10,13-dimethyl-2,3,4,5,9,11,12,15,16,17-decahydro-1H-cyclopenta[a]phenanthren-6-on;
- **Nr. 3:** (2S,3R,5R,10R,13R,14S,17S)-2,3,14-Trihydroxy-17-[2-(4-hydroxy-1-piperidyl)acetyI]-10,13-dimethyl-2,3,4,5,9,11,12,15,16,17-decahydro-1H-cyclopenta[a]phenanthren-6-on;
- **Nr. 4:** (2S,3R,5R,10R,13R,14S,17S)-2,3,14-Trihydroxy-17-[2-[4-(2-hydroxyethyl)-1-piperidyl]acetyl]-10,13-dimethyl-2,3,4,5,9,11,12,15,16,17-decahydro-1H-cyclopenta[a]phenanthren-6-on;
- **Nr. 5:** (2S,3R,5R,10R,13R,14S,17S)-17-[2-(3-Dimethylaminopropyl(methyl)amino)acetyl]-2,3,14-trihydroxy-10,13-dimethyl-2,3,4,5,9,11,12,15,16,17-decahydro-1H-cyclopenta[a]phenanthren-6-on;
- **Nr. 6:** 2-[2-Oxo-2-[(2S,3R,5R,10R,13R,14S,17S)-2,3,14-trihydroxy-10,13-dimethyl-6-oxo-2,3,4,5,9,11,12,15,16,17-decahydro-1H-cyclopenta[a]phenanthren-17-yl]ethyl]ethylsulfanylacetat;
- **Nr. 7:** (2S,3R,5R,10R,13R,14S,17S)-17-(2-Ethylsulfanylacetyl)-2,3,14-trihydroxy-10,13-dimethyl-2,3,4,5,9,11,12,15,16,17-decahydro-1H-cyclopenta[a]phenanthren-6-on;
- **Nr. 8:** (2S,3R,5R,10R,13R,14S,17S)-2,3,14-Trihydroxy-17-[2-(2-hydroxyethylsulfanyl)acetyl]-10,13-dimethyl-2,3,4,5,9,11,12,15,16,17-decahydro-1H-cyclopenta[a]phenanthren-6-on.

## Claims

1. Composition comprising at least one phytoecdysone and/or at least one semi-synthetic derivative of 20-hydroxyecdysone, for its use in inducing bronchodilation and/or in preventing or reducing exacerbated bronchial reactivity, in a mammal having an inflammatory respiratory disease, said semi-synthetic derivative of 20-hydroxyecdysone being chosen from:
- a compound of general formula (I): 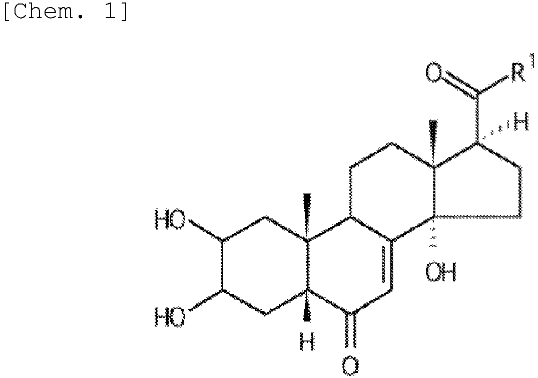 wherein:
∘ **R¹** is chosen from: a (C₁-C₆)W(C₁-C₆) group; a (C₁-C₆)W(C₁-C₆)**W**(C₁-C₆) group; a (C₁-C₆)**W**(C₁-C₆)CO₂(C₁-C₆) group; a (C₁-C₆)**A** group, **A** representing a heterocycle optionally substituted by an OH, OMe, (C₁-C₆), N(C₁-C₆) , CO₂(C₁-C₆) type group; a CH₂Br group;
**W** being a heteroatom chosen from N, O and S, preferably O and even more preferably S; and,
- a compound having formula (II):

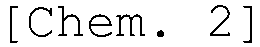

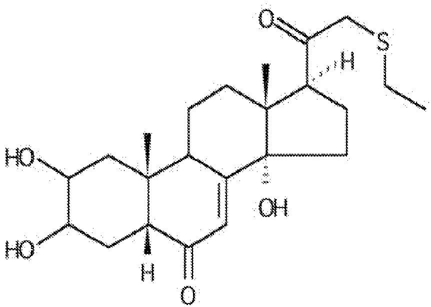

2. Composition for its use according to claim 1, wherein the inflammatory respiratory disease is a chronic inflammatory respiratory disease.

3. Composition for its use according to any one of claims 1 to 2, wherein the inflammatory respiratory disease is asthma, chronic obstructive pulmonary disease or cystic fibrosis.

4. Composition for its use according to any one of claims 1 to 3, wherein the composition is used in the treatment of respiratory function impairment linked to the serotoninergic pathway in the mammal having an inflammatory respiratory disease.

5. Composition for its use according to claim 4, wherein the composition is used in the treatment of respiratory function impairment linked to the serotoninergic pathway 5-HT_{1A} receptor in the mammal having an inflammatory respiratory disease.

6. Composition for its use according to any one of claims 1 to 5, comprising 20-hydroxyecdysone.

7. Composition for its use according to claim 6, wherein 20-hydroxyecdysone is in the form of an extract of a plant or a plant part, said plant being chosen from plants containing at least 0.5% of 20-hydroxyecdysone by dry weight of said plant, said extract comprising at least 95%, and preferably at least 97%, of 20-hydroxyecdysone.

8. Composition for its use according to claim 7, remarkably comprising between 0 and 0.05%, by dry weight of the extract, of impurities capable of affecting the safety, availability or efficacy of a pharmaceutical application of said extract.

9. Composition for its use according to any one of claims 7 to 8, wherein the plant is chosen from *Stemmacantha carthamoides, Cyanotis arachnoidea, Cyanotis vaga* and *Pfaffia paniculata*.

10. Composition for its use according to any one of claims 1 to 9, wherein in general formula (I):
**R¹** is chosen from: a (C₁-C₆)**W**(C₁-C₆) group; a (C₁-C₆)**W**(C₁-C₆)**W**(C₁-C₆) group; a (C₁-C₆)**W**(C₁-C₆)CO₂(C₁-C₆) group; a (C₁-C₆)**A** group, **A** representing a heterocycle optionally substituted by an OH, OMe, (C₁-C₆), N(C₁-C₆), CO₂(C₁-C₆) type group;
**W** being a heteroatom chosen from N, O and S, preferably O and more preferably S.

11. Composition for its use according to any one of claims 1 to 10, wherein said compound of general formula (I) is chosen from:
- **No. 1:** (2S,3R,5R,10R,13R,14S,17S)-2,3,14-trihydroxy-10.13-dimethyl-17-(2-morpholinoacetyl)-2,3,4,5,9,11,12,15,16,17-decahydro-1H-cyclopenta[a]phenanthren-6-one;
- **No. 2:** (2S,3R,5R,10R,13R,14S,17S)-2,3,14-trihydroxy-17-[2-(3-hydroxypyrrolidin-1-yl)acetyl]-10,13-dimethyl-2,3,4,5,9,11,12,15,16,17-decahydro-1H-cyclopenta[a]phenanthren-6-one;
- **No. 3:** (2S,3R,5R,10R,13R,14S,17S)-2,3,14-trihydroxy-17-[2-(4-hydroxy-1-piperidyl)acetyI]-10,13-dimethyl-2,3,4,5,9,11,12,15,16,17-decahydro-1H-cyclopenta[a]phenanthren-6-one;
- **No. 4:** (2S,3R,5R,10R,13R,14S,17S)-2,3,14-trihydroxy-17-[2-[4-(2-hydroxyethyl)-1-piperidyl]acetyl]-10,13-dimethyl-2,3,4,5,9,11,12,15,16,17-decahydro-1H-cyclopenta[a]phenanthren-6-one;
- **No. 5:** (2S,3R,5R,10R,13R,14S,17S)-17-[2-(3-dimethylaminopropyl(methyl)amino)acetyl]-2,3,14-trihydroxy-10,13-dimethyl-2,3,4,5,9,11,12,15,16,17-decahydro-1H-cyclopenta[a]phenanthren-6-one;
- **No. 6:** 2-[2-oxo-2-[(2S,3R,5R,10R,13R,14S,17S)-2,3,14-trihydroxy-10,13-dimethyl-6-oxo-2,3,4,5,9,11,12,15,16,17-decahydro-1H-cyclopenta [a]phenanthren-17-yl] ethyl] ethy 1 sulfanylacetate;
- No. 7: (2S,3R,5R,10R,13R,14S,17S)-17-(2-ethylsulfanylacetyl)-2,3,14-trihydroxy-10.13-dimethyl-2,3,4,5,9,11,12,15,16,17-decahydro-1H-cyclopenta[a]phenanthren-6-one;
- **No. 8:** (2S,3R,5R,10R,13R,14S,17S)-2,3,14-trihydroxy-17-[2-(2-hydroxyethylsulfanyl)acetyl]-10,13-dimethyl-2,3,4,5,9,11,12,15,16,17-decahydro-1H cyclopenta[a]phenanthren-6-one.
